# EUROPEAN PATENT APPLICATION

(11) **EP 2 474 605 A1**
(43) Date of publication of application: **11.07.2012**
(21) Application number: 11165393.7
(22) Date of filing: 10.05.2011
(51) Int. Cl.: C12M 1/00

(54) **Container fermentor**

(30) Priority: 11.01.2011 SK 500032011 U
(71) Applicant: KHT s.r.o., 058 01 Poprad (SK)
(72) Inventor: Bencic, Mario, SK-058 01, Poprad (SK); Rusin, Jan, SK-058 01, Poprad (SK)
(74) Representative: Bachrata, Magdaléna

(57) **Abstract**

The container fermentor for biomass processing consists of the multilayer insulation cover (1), the waterproof and an airproof door for loading and unloading of the biomass and the control unit (3), connected to the sensors (2) of parameters of a fermentation, all placed inside the fermentor. Internal sides are equipped with heating elements (6) and the top of the internal side is equipped with spray nozzles (11), that are connected to the container (4), whereas fermentor includes at least one chamber (8), consisting of the frame (12), the floor (15), side walls (16) and the front wall (17). The floor (15), the side walls (16) and the front side (17) may be filled in by the network (19). The chamber (8) may be equipped on the frame (12) by pivots (14) for unloading, at the bottom side of the frame (12) may be fitted the mobile element (13) and in the corners drawing wheels (18).

## Description

### Field of the invention

The invention relates to a container fermentor (reactor) for a biomass processing with an assistance of a dry fermentation in an anaerobic environment that is a mobile one, is adapted to a carrying and a manipulation and its construction simultaneously allows for a parallel connection of more fermentor containers into a colony.

### Background of the invention

At the present time a biologically decomposable biomass is processed in apparatuses using aerobic processes like aeration. These apparatuses represent composting piles, barriers, canals etc. A disadvantage of such a system lies in a non-exploitation of the produced gasses [methane (CH₄), carbon dioxide (CO₂)] that participate in the process of a global warming, in the so called "greenhouse effect", in a need of a great manipulation area, in creation of stench, attracting rodents and insects and in taking a long processing time.

The biomass is further processed in apparatuses using anaerobic bacteria during wet or dry processes. The document DE 199 58 142 A1 presents a bioenergic station where the fermentor is placed in its construction part and is firmly connected with the ground. This station does not tackle the problem of the biomass loading.

The leaflet of Claus Rückert Office, Biogasanlagen, 92259, Neukirchen, shows a solution for the biomass processing that requires a construction of two separate buildings where fermentors are placed in one of these buildings.

The bioenergic station according to DE 197 46 636 A1 is partially embedded into the ground and consists of four separate buildings in which fermentors are placed.

The document EP 1 069 174 A1 discloses a gas generator run by a biogas that is placed outside the building and the fermentor that is placed in the building firmly connected with ground. The heat produced from combustion gasses allows for biomass dehydration.

The disadvantage of such systems stems in its large built-up and manipulation area, construction parts firmly connected by the base to the ground, expensive construction and technical adjustments for providing a waterproof and airproof constructions, precise recipe dosing during the fermentor loading, absence of the whole process management, problems relating to the container storing of the centrifuge and its consecutive application as a fertilizer into a soil, where most of all, the water protection from the agricultural sources nitrate contamination is concerned.

Document BE 1017711 A6 describes a transferable unit for biogas production in a shape of a container that is able to be transported long distances and is easily adjustable when on a place. It is a container used for a wet fermentation, wherein biomass is mixed into water and is left to undergo a fermentation process. Then, it is released through the valves into stationary centrifuge intercontainers or directly into a sewage treatment plan.

A disadvantage of the mentioned solution is the impossibility to fill in the container with usual gadgetry, that the process is not controlled and that mixing is provided only by the means of a centrifugate flowing from one chamber to another.

### Subject-matter of the invention

Mentioned disadvantages are eliminated by the container fermentor (reactor) for biomass production which subject matter is that it contains a multilayer insulation cover, waterproof and airproof door for loading and unloading of a biomass and a control unit connected to sensors of parameters of fermentation that are placed inside the fermentor. The whole internal side of the container fermentor is equipped with heating elements and on its upper internal side are fitted spray nozzles connected to the container. The fermentor also includes at least one chamber consisting of a frame, a floor, side walls and a front wall that may be filled in by a network.

The container fermentor control unit provides for optimum conditions for a biochemical process.

It is of an advantage, should the chamber frame be equipped with pivots assisting in the chamber turning in 180° degrees that leads to a chamber unloading. It is also of an advantage if the travel is fitted to the bottom side of the frame and in its corners are drawing wheels assisting to an easier insertion of the chamber into the container fermentor. The travel of the mobile chamber is equipped with wheels, eventually also ski may be used depending on the way of the chamber insertion into the container fermentor.

Specified construction of the chamber allows for penetration of the percolate into the biomass and simultaneously it allows for biogas leakage through the whole surface.

According to the mentioned technical solution an advantage of the container fermentor lies in its mobility and that it allows for biogas and fermented substrate production with assistance of biomass processing in an anaerobic environment. Further advantage is that it allows for a simple and a quick manipulation of a biomass during loading and unloading as well as it allows controlling the whole fermentation process.

### Brief description of the drawings

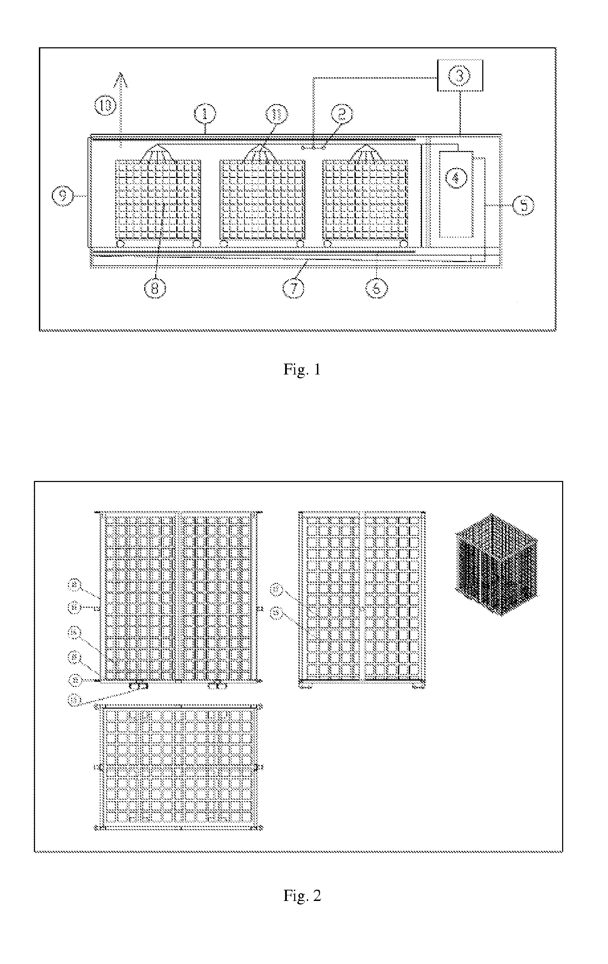

Technical solution is explained in a detail on a set of figure. Fig. 1 shows a fermentation container with a description of main construction parts. Fig. 2 shows a chamber with a description of main parts according to this solution.

### Detailed description of an example

The container fermentor on Fig. 1 is an apparatus for the biomass anaerobic processing constructed from the multilayer insulation cover 1 that protects the fermentation process from non-appropriate atmospheric conditions and the waterproof and airproof (gasproof) door 9 of the whole container fermentor. The waterproof and airproof door 9 allows for entering the container fermentor and manipulation within it as loading and unloading of the chamber 8, service interferences, maintaining and cleaning of heating elements 6. The control unit 3 is a component of the container fermentor, which with an assistance of sensors 2 of parameters of fermentation collects data, evaluates them and controls the fermentation process. Along the internal side of the fermentation container are fitted heating units 6, assisting the process to be kept in an optimal temperature. Spray nozzles 11 are placed over each chamber and serve to moisten the mass by the percolate 5 from the container 4 filled in by pumping off the percolate 5 from the fermentor floor 7. Exhausted biogas 10 from the container fermentor is transferred to storage or further processing. Loading of the container fermentor is being done through chambers 8 made out of the frame 12, the floor 15, side walls 16 and the front wall 17 and may be filled in by the network 19 from a drawing metal. Chambers 8 are made of the material resistant to the aggressive environment that is created by fermentation. Chambers 8 have a wheel mobile element 13 on the bottom. For the purpose of unloading, pivots 14 are fitted on the frame assisting in the chamber turning in 180° degrees that leads to the chamber unloading. In the chamber 8 corners are placed drawing wheels 18 assisting to an easier insertion of the chamber into the container fermentor.

A producer of biological raw materials and/or biologically decomposing communal waste is able to process all produced biological raw materials and/or biologically decomposing communal waste in a way that such material is filled in chamber 8 of the container fermentor for non-waste biomass processing in an anaerobic environment. The chamber 8 is then inserted into the container fermentor and this one is waterproof and airproof closed. In the container fermentor the control process of fermentation begins. The process consists of the macromolecule partition caused by the hydrolytic bacteria; the fermentation of the products of partition caused by acidogenous bacteria, the creation of methanogennous substrates by acetogennous bacteria and also consists of a gas creation caused by methanogennuous bacteria. The result of the fermentation process is fermentised substrate and a biogas 10 consisting of methane (CH₄), carbon dioxide (CO₂) and of a small amount of ingredients. Produced biogas 10 is being exhausted in the process of fermentation and it is used for further use. Fermentation control process in the container fermentor is provided by the control unit 3. It scans data concerning temperature, pressure, pH and an amount of CH₄ and CO₂ in the biogas 10. Based on acquired data it controls a temperature via an amount of a warm water in a heating system, it controls the pressure via biogas 10 exhaustion, used for further processing, and it controls pH through an amount of sprayed percolate 5. The amount of CH₄ a CO₂ is being monitored for the needs of the further use.

### Industrial applicability

The container fermentor is applicable for a non-waste biomass processing (biological raw material and/or biologically decomposing communal waste) in an anaerobic environment.

### List of reference marks

- 1.: multilayer insulation cover
- 2.: sensors of a parameters of fermentation
- 3.: control unit
- 4.: container
- 5.: percolate
- 6.: heating elements
- 7.: fermentor floor
- 8.: chamber
- 9.: water-proof and air-proof door
- 10.: biogas
- 11.: spray nozzles
- 12.: frame
- 13.: mobile element
- 14.: pivot
- 15.: floor
- 16.: side wall
- 17.: front wall
- 18.: drawing wheel
- 19.: network

## Claims

1. A container fermentor consisting of a multilayer insulation cover (1), waterproof and airproof door (9) **characterized in that** it contains heating elements (6) on the internal sides, on the top of an internal side is equipped with spray nozzles (11) connected to a container (4) and a control unit (3) connected to sensors (2) of parameters of fermentation placed inside the fermentor, whereas fermentor includes at least one chamber (8), consisting of a frame (12), floor (15), side walls (16) and a front wall (17) are filled in by a network (19).

2. The container fermentor according to claim 1 **characterized in that** the floor (15), the side walls (16) and the front wall (17) are filled in by the network (19).

3. The container fermentor according to claim 1 or 2, **characterized in that** the frame (12) is equipped with pivots (14) for unloading of the chamber (8).

4. The container fermentor according to any of claims 1 to 3, **characterized in that** the mobile element (13) is located at the bottom side of the frame (12).

5. The container fermentor according to claim 4, **characterized in that** the mobile element (13) is equipped with wheels.

6. The container fermentor according to claim 4, **characterized in that** the mobile element (13) is equipped with a ski.

7. The container fermentor according to any of claims 1 to 4, **characterized in that** the drawing wheels (18) are in the corners of the frame (12).
